# EUROPÄISCHE PATENTSCHRIFT

(11) **EP 0 991 587 B1**
(45) Veröffentlichungstag und Bekanntmachung des Hinweises auf die Patenterteilung: **22.09.2004**
(21) Anmeldenummer: 98934995.6
(22) Anmeldetag: 24.06.1998
(51) Int. Cl.: C01B 3/38

(54) **VERFAHREN UND VORRICHTUNG ZUR AUTOTHERMEN REFORMIERUNG VON KOHLENWASSERSTOFFEN**
METHOD AND DEVICE FOR REFORMING HYDROCARBONS AUTOTHERMALLY
PROCEDE ET DISPOSITIF DE REFORMAGE AUTOTHERME D'HYDROCARBURES

(30) Priorität: 24.06.1997 DE 19727841
(43) Veröffentlichungstag der Anmeldung: 12.04.2000
(73) Patentinhaber: Fraunhofer-Gesellschaft zur Förderung der angewandten Forschung e.V., 80686 München (DE)
(72) Erfinder: MAIER-RÖLTGEN, Uli, D-79379 Müllheim (DE); SCHULER, Alexander, D-79098 Freiburg (DE); FINKBEINER, Hartmut, D-72270 Baiersbronn (DE)
(74) Vertreter: Pfenning, Meinig & Partner
(86) Internationale Anmeldenummer: PCT/EP1998/003869
(87) Internationale Veröffentlichungsnummer: WO 1998/058874

(56) Entgegenhaltungen:
- WO-A-92/00241
- GB-A- 2 153 382
- GB-A- 2 222 096
- GB-A- 2 222 533

## Beschreibung

Die Erfindung betrifft eine Vorrichtung und ein Verfahren zur autothermen Reformierung von Kohlenwasserstoffen, die beispielsweise in Form von Erdgas, Benzin, Methanol, Diesel, Flüssiggas usw. vorhanden sind.

Die industrielle Wasserstofferzeugung aus fossilen Energieträgern wie Erdgas, Flüssiggas oder Naphta wird hauptsächlich nach dem Steam-Reforming-Verfahren in mit Katalysator befüllten Röhrenöfen mit indirekter Beheizung durchgeführt. Die wasserstoffreichen Synthesegase dienen beispielsweise der Herstellung von Ammoniak, Alkoholen oder zur Methanolsynthese, aber auch zur Gewinnung von Reinstwasserstoff. Bedingt durch die Größe der Anlagen sind aufwendige Verfahrensschritte in getrennten Reaktorkonstruktionen möglich.

Neben den großindustriellen Wasserstofferzeugungsanlagen sind im Zuge der Brennstoffzellenforschung spezielle Reformer entwickelt worden, die auf dem sogenannten heat-exchange-Prinzip basieren. Die Produktionsleistung an Wasserstoff liegt bei diesen Reformern in der Größenordnung einiger 100 kW bezogen auf den unteren Heizwert des Wasserstoffvolumenstroms. Als Wasserstoffnutzer werden die Hochtemperaturbrennstoffzellen SOFC oder MCFC und die bereits kommerziell vertriebene Phosphorsaure Brennstoffzelle zur dezentralen Stromerzeugung eingesetzt.

Für kleinere Systeme mit Polymermembranbrennstoffzellen wird im Hinblick auf die mobile Anwendung meist Methanol als Energieträger gewählt, wobei die autotherme Reformierung von Methanol schon bei Temperaturen von 200°C bis 300° C abläuft und gegenüber der Reformierung von z.B. Methan eine geringere Reaktionsenthalpie erfordert.

Die obengenannten heat-exchange-Reformer zeichnen sich durch Gegenstrom- und Gleichstromführung der Stoffströme Rauchgas und Prozeßgas aus. Die Reformierreaktion findet in mit Feststoffkatalysator, zum Beispiel Ni/Al₂O₃ gefüllten Rohr- oder Ringspaltreaktoren statt. Der Flammenbrenner liegt meist zentrisch in der Reformiereinheit oder ist an den Reformer angeflanscht. Die Rauchgase werden dann in Spalten an den Reaktorwänden vorbeigeführt, wo der für die Reaktion benötigte Wärmestrom konvektiv übertragen wird. Voraussetzung für gute Umsätze sind ausreichend große Übertragerflächen. Das Prozeßgas nach der Reformierung wird ebenfalls im Gegen- oder Gleichstrom zum eintretenden Eduktgas geführt, um den hohen Enthalpiestrom des Synthesegases wenigstens teilweise zur Aufwärmung der Edukte zu nutzen und damit Feuerungsleistung zu sparen. Die Nachteile der bestehenden Reformer sind darin zu sehen, daß sie nicht durch einfaches Scale down auf kleine Systeme angepaßt werden können und insbesondere das thermische Management gestaltet sich in kleinen Reaktoren schwierig.

Aus der WO 92/00241 ist ein Verfahren und eine Vorrichtung zur Reformierung von Kohlenwasserstoffen bekannt, bei denen einem autothermen Reaktor ein Gemisch aus Brennstoff und Ausgangsstoffen der Reformierung zugeführt wird, das vorher auf hohe Temperaturen zwischen 800° und 1600° aufgeheizt wird.

Die GB-A-222 096 und GB-A-222 533 beschreiben ein zweistufiges Verfahren, d.h. ein allothermes und autothermes Verfahren mit einem primären und einem sekundären als Röhren ausgebildeten Reformierer, bei dem das ein Kohlenwasserstoff und Wasserdampf aufweisende Gemisch dem primären Reformierer zugeführt wird, wobei die Reformierung im sekundären Reformierer unter Zuführung von Sauerstoff fortgesetzt wird.

Der Erfindung liegt die Aufgabe zugrunde, ein Verfahren und eine Vorrichtung zur autothermen Reformierung von Kohlenwasserstoffen insbesondere im kleinen Leistungsbereich zu schaffen, die sowohl für gasförmige als auch flüssige Brennstoffe geeignet sind, wobei unerwünschte Reaktionen, wie Rußbildung, Verkokung oder thermisches Cracken vermieden werden und ein gutes dynamisches Verhalten geliefert wird und wobei insbesondere für die mobile Anwendung die Vorrichtung klein und kompakt ausgebildet sein soll.

Diese Aufgabe wird erfindungsgemäß durch die Merkmale des Hauptanspruchs und des nebengeordneten Anspruchs gelöst.

Dadurch, daß der Brennstoff, gemischt mit Ausgangsstoffen der Reformierung direkt auf den heißen Katalysator des Reformierreaktors aufgebracht wird und die Verbrennung und Reformierung im Wesentlichen in dem gleichen Reaktionsbereich stattfindet, werden unerwünschte Reaktionen, wie thermisches Cracken, Verkokung, Rußbildung weitgehend vermieden, da der insbesondere bei flüssigen Kohlenwasserstoffen kritische Temperaturbereich von etwa 250°C bis 600°C schnell durchlaufen wird. Dies wird durch die Gemischaufbereitung (Brennstoff-Luft-Wasserdampf) im Reaktor unterstützt, wobei durch den heißen Katalysator das Gemisch extrem schnell erwärmt wird.

Es können mit ein und derselben Reaktorkonstruktion sowohl gasförmige als auch flüssige Brennstoffe und auch höhere Kohlenwasserstoffe, wie Benzin oder Diesel reformiert werden.

Aufgrund der Möglichkeit, den Kohlenwasserstoff in bereits vorgewärmte Luft und eventuell Wasserdampf einzudüsen bzw. schon mit vorgewärmter Luft und/oder Wasserdampf zu verdüsen oder beides und aufgrund der Erwärmung der Edukte insbesondere der Flüssigkeitströpfchen des verdüsten Kohlenwasserstoffs durch Wärmerückstrahlung des heißen Katalysators in die Einlaß- und Mischzone zwischen Düse und Katalysator ist ein schnelles Verdampfen und Erwärmen, insbesondere von flüssigen Kohlenwasserstoffen, gegeben, so daß der Kohlenwasserstoff extrem schnell auf Prozeßtemperaturen gebracht wird und die Reaktion sofort in die gewollte Richtung verlaufen kann.

Vorzugsweise wird mindestens ein Teil der Ausgangsstoffe in einem Wärmetauscher im Wärmetausch mit dem aus dem Reformierreaktor austretenden Reformat vorgeheizt.

Zum Starten der Vorrichtung wird eine kurze Startzeit durch geringe aufzuheizende Masse ermöglicht, die Temperatur des Katalysators ist durch Zugabe der beispielsweise als Luft ausgebildeten Ausgangsstoffe frei einstellbar und die Wasserstoffausbeute ist durch Zugabe von Wasser oder Wasserdampf steuerbar. Insgesamt wird eine hohe Leistungsdichte pro Systemvolumen erreicht. Darüber hinaus wird eine einfache und kompakte integrative Bauweise zur Verfügung gestellt, da ein separater Verdampfer oder eine Verdampferstufe für flüssige Brennstoffe wegfällt, und keine externe Beheizung, das heißt ein Brenner notwendig ist.

Weiterhin ist eine Umsetzung von verschiedenen Kohlenwasserstoffen in einem Gerät möglich und es kann der Betrieb sowohl unter Druck als auch drucklos durchgeführt werden. Die Kohlenwasserstoffmenge kann in weiten Bereichen (1:5 und mehr) moduliert werden und es sind sehr kleine Leistungen möglich.

Es wird eine autotherme Reformierung in einem Wabenkatalysator und/oder in einer Katalysatorschüttung durchgeführt, wobei die Reaktion bei relativ niedrigen Temperaturen ermöglicht wird und Materialprobleme vermieden werden. Eine vorherige Verdampfung des flüssigen Kohlenwasserstoffs ist nicht notwendig. Durch Vorsehen des Wärmetauschers um die Reaktionszone können die als Ausgangsstoffe verwendete Luft und/oder der Sauerstoff und/oder das Wasser und/oder der Wasserdampf vorgeheizt werden und es werden Wärmeverluste nach außen vermieden. Es ist eine Zugabe von Luft und/oder Sauerstoff und/oder Wasser und/oder Wasserdampf über die Zufuhrdüse, die als Zweistoffdüse ausgebildet sein kann, und den Wärmetauscher möglich. Die Regelung der Prozeßbedingungen kann durch Einstellung der Verhältnisse der Zugabe leicht vorgenommen werden. Weiterhin kann das Wasser bzw. der Wasserdampf auch gemeinsam mit dem Kohlenwasserstoff über die Düse zugegeben werden, wodurch Rußbildung und Ablagerungen unterdrückt werden.

Durch die in den Unteransprüchen angegebenen weiteren Maßnahmen sind vorteilhafte Weiterbildungen und Verbesserungen möglich.

Ausführungsbeispiele der Erfindung sind in der Zeichnung dargestellt und werden in der nachfolgenden Beschreibung näher erläutert. Es zeigen:
- Fig. 1: einen Schnitt durch ein erstes Ausführungsbeispiel der erfindungsgemäßen Vorrichtung,
- Fig. 2: einen Schnitt durch ein zweites Ausführungsbeispiel der erfindungsgemäßen Vorrichtung,
- Fig. 3: einen Schnitt durch ein drittes Ausführungsbeispiel der erfindungsgemäßen Vorrichtung, und
- Fig. 4: einen Schnitt durch ein viertes Ausführungsbeispiel der Erfindung.

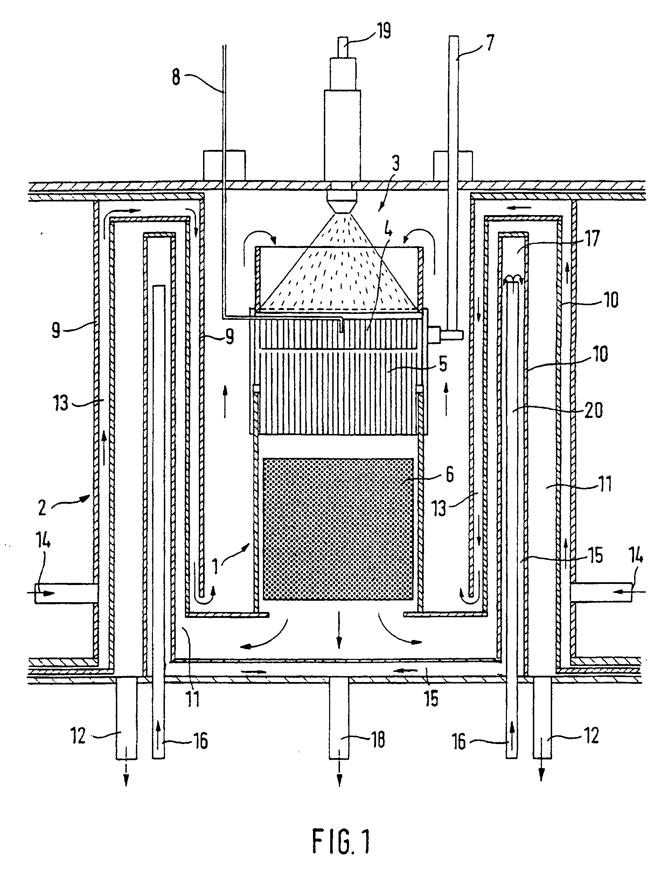

In Fig. 1 ist eine Vorrichtung zur autothermen Reformierung dargestellt, die flüssige und/oder gasförmige Kohlenwasserstoffe katalytisch in Synthesegase umsetzt. Die Vorrichtung besteht aus einem Reformierreaktor 1, einem Wärmetauscher 2 und einem Eindüsungs-Mischungsraum 3, die in einer Konstruktion bzw. in einer Einheit realisiert sind. Der Reformierreaktor 1 besteht aus zwei Bereichen, einem oberen Bereich und einem unteren Bereich, wobei der obere Bereich Wabenkörper 4, 5 und der untere Bereich eine Schüttung 6 aufweist. Es sind ein erster Wabenkörper 4 und ein davon getrennter zweiter Wabenkörper 5 vorgesehen, wobei die Wabenkörper aus Metall oder Keramik bestehen können, die als Träger für einen Katalysator dienen. Die Wabenkörper sind daher mit einem Katalysator beschichtet, der aus Platin besteht oder Nickel aufweist, wobei jedoch jegliche Katalysatorbeschichtung verwendet werden kann, sofern diese für die Reformierung geeignet ist. Gleiches gilt für den Katalysatorträger. Die Schüttung ist im vorliegenden Ausführungsbeispiel als keramische Schüttung ausgebildet, die ebenfalls mit Katalysator entsprechend den obigen Angaben beschichtet ist.

Anstelle der Schüttung kann auch ein Wabenkörper bzw. ein entsprechender mit Katalysatormaterial versehener permeabler Festkörper verwendet werden, bei dem der Druckverlust geringer ist.

Im oberen Bereich der Vorrichtung ist eine als Düse 19 ausgebildete Zufuhrvorrichtung für Brennstoff vorgesehen, die in den Mischraum 3 mündet, wobei die Düse 19 als Ein- oder vorzugsweise als Zweistoffdüse ausgebildet ist. Zusammen mit dem Brennstoff kann sowohl die Luft, Sauerstoff, Wasserdampf als auch das Wasser miteingebracht werden, wobei der entsprechende Ausgangsstoff kalt oder über den Wärmetauscher vorgewärmt ist.

Wesentlicher Grundgedanke des Verfahrens zur autothermen Reformierung von Kohlenwasserstoffen, das mit einer Vorrichtung nach Fig. 1 durchgeführt werden kann, ist das Aufdüsen eines Brennstoff-Luft- bzw. Gas- und/oder Wasserdampf-Gemisches mit Temperaturen von bis zu 250° C auf einen auf z.B. 600°C oder mehr erhitzten Katalysator, wodurch bei flüssigem Brennstoff durch die Strahlungswärme beim Aufdüsen entstandene Brennstofftröpfchen schon teilweise verdampfen, bevor sie auf den Katalysator treffen. Die Umsetzung des Brennstoffgemisches findet in dem Katalysator zu jeder Zeit rein katalytisch, also flammenlos statt.

Die Aufheizung des Brennstoffgemisches auf die gewünschte Aufbringungstemperatur kann über den Wärmetauscher durchgeführt werden.

Zum Starten des Betriebes des Reaktors 1 wird der erste Katalysator 4 elektrisch beheizt, was durch die elektrische Leitung 7 angedeutet ist. Anstelle oder zusätzlich zu der elektrischen Heizung kann im Mischraum 3 eine Zündvorrichtung vorgesehen sein. In der Startphase wird der Brennstoff zusammen mit zugeführter Luft zur Verbrennung und Aufheizung des Katalysators auf die gewünschte Reaktionstemperatur verwendet. Im darauffolgenden Reformierbetrieb wird durch Regelung der zugeführten Stoffe die Temperatur gehalten.

Im Bereich des ersten Wabenkörpers 4 ist ein Temperatursensor mit Anschlußleitung 8 vorgesehen, der zur Überwachung der Temperatur dienen.

Der Wärmetauscher 2 besteht aus mehreren zylindrischen Wänden 9 bzw. zylindrischen ringförmigen Hohlkörpern 10, die so angeordnet und ineinander verschachtelt sind, daß sie mindestens zwei getrennte Strömungswege mit mehrfachen Umleitungen bilden. Ein Strömungsweg 11 für das Reformat schließt sich an das untere Ende des Reformierreaktors 1, an dem das Reformat austritt, an, wird nach oben und anschließend nach unten umgelenkt und mündet in einen Auslaß 12.

Ein weiterer Strömungsweg 13 nach Fig. 1 steht mit einem Eintrittsanschluß 14 für Luft, Sauerstoff, Wasser und/oder Wasserdampf in Verbindung, wird im oberen Bereich der Vorrichtung nach unten umgelenkt und mündet in den freien Raum zwischen Wärmetauscher 2 und Reformierreaktor 1, in dessen oberem Bereich sich der Mischraum 3 befindet. Weiterhin wird ein abgeschlossener Strömungsweg 15 gebildet, der mit einem Einlaß 16 gleichfalls für Luft, Sauerstoff, Wasser oder Wasserdampf verbunden ist, und daran anschließend von einem hohlen Ringrohr 17 begrenzt ist, am Austritt des Ringrohrs 17 nach unten umgelenkt wird und am Auslaß 18 aus dem Wärmetauscher herausführt. Im Ausführungsbeispiel ist der bzw. sind die Einlässe 16 mit jeweils einem in das Ringrohr 17 hineinragende Hohlrohr 20 verbunden, von denen über den Umfang mehrere vorgesehen sein können. Der Strömungsweg 15 dient zur Vorerwärmung des Ausgangsstoffes und sein Auslaß 18 kann mit dem Einlaß 14 oder auch mit einer Zufuhreinrichtung im oberen Bereich der Vorrichtung verbunden sein.

Der Wärmetauscher 2 ist, wie das Ausführungsbeispiel zeigt, um den Reaktionsraum herum angeordnet, wobei er aus konzentrischen Rohren besteht und in den dadurch entstehenden Ringspalten die Ausgangsstoffe der Wärme, d.h. die zugeführte Luft, der Sauerstoff, der Wasserdampf und eventuell das zugeführte Wasser im Gegenstrom zum Reformat im Wärmetausch mit diesem von außen nach innen geführt werden. Dadurch werden die Wärmeverluste nach außen minimiert und aufwendige, der kompakten Bauweise abträgliche Isolationsmaßnahmen vermieden. Der Aufbau des Reformierreaktors 1 und des Wärmetauschers 2 muß, wie in der Ausgestaltung gezeigt, Wärmedehnungen ohne unzulässige Verspannungen zulassen, um die Dichtheit und Materialbeständigkeit des Reformers zu gewährleisten. Dies wird dadurch erreicht, daß im heißen Mittelbereich die Ringrohre hängend angeordnet sind und die Teile des Wärmetauschers nur außen im kalten Bereich zum Beispiel verschraubt sind.

Zu Beginn des Betriebes der Vorrichtung wird der erste Wabenkörper 4 vorgeheizt und der Brennstoff in flüssiger und/oder gasförmiger Form über die Düse 19 zusammen mit dem Ausgangsstoff, der vorgewärmt sein kann, d.h. Luft und/oder Sauerstoff gegebenenfalls Wasserdampf in den Mischraum 3 eingeführt und direkt auf den Wabenkörper 4 aufgedüst. Dadurch wird die Verbrennung des Brennstoffs gestartet. Nach Erreichen der nötigen Reformierungstemperatur wird durch Ändern der Luftzufuhr bzw. Sauerstoffzufuhr und Zugabe von Wasser oder Wasserdampf in den Reformierungsbetrieb übergegangen, bei dem Reformierung und Verbrennung parallel ablaufen. Die Verdüsung mit Wassserdampf bewirkt einen besseren Brennstoffumsatz und minimiert weiterhin eine eventuelle Rußbildung. In dem Reformierreaktor wird die katalytisch unterstützte Verbrennung und die Reformierung selbst gleichzeitig katalytisch durchgeführt.

Über den Sensor 8 kann die Temperatur überwacht werden, wobei abhängig von dem Meßwert die Temperatur angepaßt werden kann. Während des Betriebes können die Temperatur, der Stoffdurchsatz und die Temperaturverteilung im Katalysator gezielt beeinflußt werden. Die freien Parameter sind der Luftdurchsatz (Sauerstoffdurchsatz), der Brennstoffdurchsatz und der Wasserdurchsatz, sowie die Zugabe der Luft und des Wassers direkt ohne Vorwärmung in die Reaktionszone oder indirekt ganz oder teilweise über den Wärmetauscher in die Reaktionszone. Dadurch ist eine hohe Modulierbarkeit mit annähernd gleichbleibender Produktgaszusammensetzung möglich. Besonders in Verbindung mit Brennstoffzellen, die ein exzellentes Teillastverhalten besitzen und dort auch betrieben werden, stellt die Flexibilität des Reformers ein wichtiges Kriterium dar.

Das Reformat tritt, wie durch die Pfeile in Fig. 1 angedeutet, unten aus dem Schüttbett 6 aus, wird in dem Strömungsweg 11 nach oben gelenkt, im oberen Bereich wieder umgelenkt und tritt am Auslaß 12 aus der Vorrichtung aus. Gleichzeitig wird im Gegenstrom über den Einlaß 14 und den Strömungsweg 13 der Ausgangsstoff von außen nach innen in den Reaktorraum eingeleitet, wobei das Reformat seine Temperatur an die Ausgangsstoffe abgibt. Zusätzlich wird Luft, Sauerstoff, Wasserdampf oder Wasser über den Einlaß 16 in das Hohlrohr 20 eingeleitet, im oberen Bereich umgelenkt und am Auslaß 18 wieder herausgezogen, wobei die Stoffe gleichfalls durch die Wärme des Reformats aufgeheizt werden. Je nach Bedarf kann der Wärmetauscher 2 vollständig, d.h. vom Einlaß 16 über den Strömungsweg 15 zum Auslaß 18, wobei die Strömung auch umgekehrt erfolgen kann, d.h. der Einlaß ist bei 18 und der Auslaß bei 16 und dann vom Auslaß 16 bzw. 18 zum Einlaß 14 und weiter über den Strömungsweg 13, durchströmt werden. Er kann jedoch auch nur teilweise über den Einlaß 14 und den Strömungsweg 13 durchströmt werden oder bei 14 kann eine Mischung aus dem vorgewärmten Ausgangsstoff und frischem Ausgangsstoff zugeführt werden.

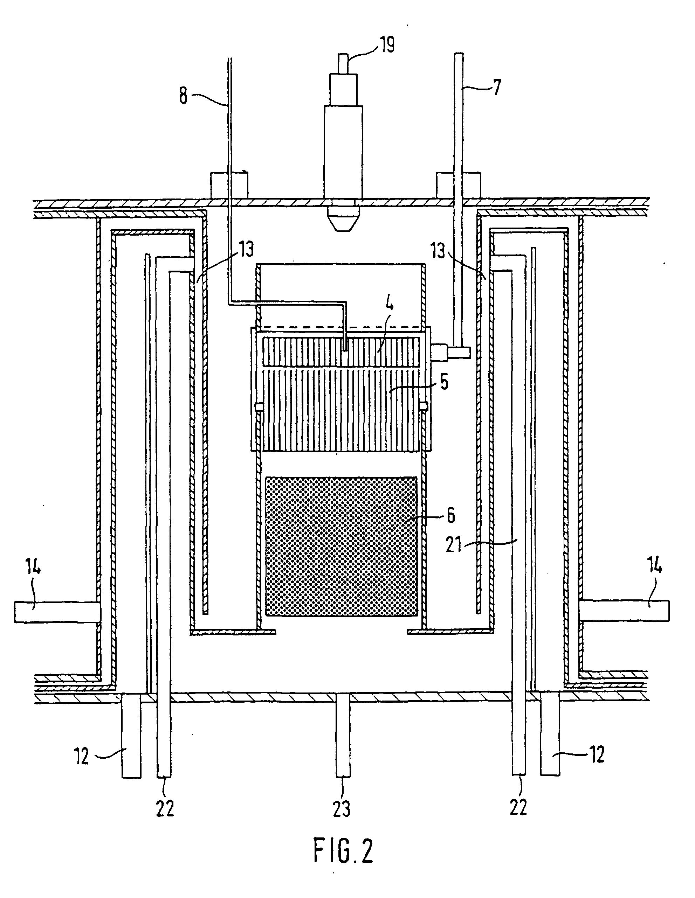

In Fig. 2 ist ein weiteres Ausführungsbeispiel dargestellt, wobei hier ein oder mehrere Rohre 21 vorgesehen sind, die im oberen Bereich der Vorrichtung im heißen Teil des Wärmetauschers 2 im Strömungsweg 13 münden. Den Rohren 21 wird beim Einlaß 22 Wasser bzw. Wasserdampf zugegeben, das sich an der heißeren Stelle des Wärmetauschers 2 mit dem über den Einlaß 14 zugeführten Ausgangsstoff vermischt. Dies ermöglicht insbesondere beim Hochfahren des Systems eine frühere Zugabe von Wasser oder Wasserdampf ohne die Gefahr des Auskondensierens und Ansammelns von Wasser im System. Über eine Zuführung 23, die als Düse ausgebildet sein kann, besteht die Möglichkeit, das Reformat sofort nach der Reformierung herunter zu quenchen, d.h. durch Wassereindüsung schneller herunterzukühlen, was eine eventuelle Kohlenstoffabscheidung unterdrückt.

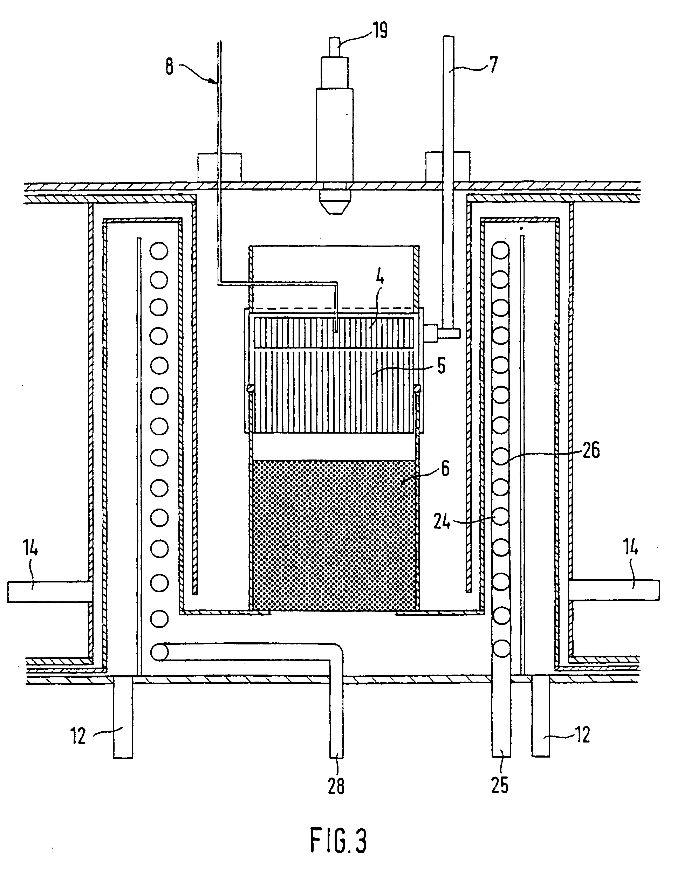

In Fig. 3 weist der Wärmetauscher 2 eine Verdampferwendel 24 auf, wobei über einen Zulaß 25 in einem Rohr 26 Wasser oder Wasserdampf in den oberen Bereich der Wendel geleitet wird, in der auf dem Weg nach unten eine vollständige Verdampfung stattfindet und bei dem Auslaß 28 kann der erhitzte Ausgangsstoff abgezogen werden, der wiederum vollständig oder teilweise dem Einlaß 14 oder vollständig oder teilweise der Düse 19 zugeführt werden kann. In der Verdampferwendel 24 kann auch Brennstoff vorbehandelt und entsprechend dem Prozeß zugeführt werden.

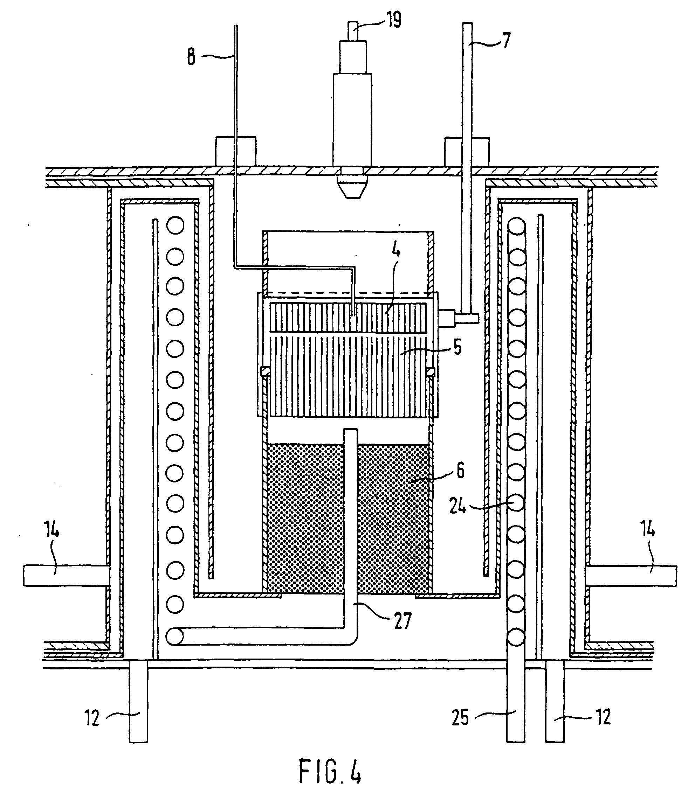

Das Ausführungsbeispiel nach Fig. 4 ist ähnlich zu dem nach Fig. 3, wobei dem Einlaß 25 der Verdampferwendel Brennstoff, Wasser bzw. Wasserdampf, Luft und/oder Sauerstoff zugegeben werden kann. Der Auslaß der Verdampferwendel 24 ist mit einer Rohrleitung 27 verbunden, die direkt in dem Raum zwischen zweitem Wabenkörper 5 und Schüttung 6 mündet. Der oder die über den Zulaß 25 eingeführten Stoffe werden nach der Vorwärmung über die Verdampferwendel 24 zwischen den beiden Katalysatoren, bestehend aus beschichtetem Wabenkörper 5 und beschichteter Schüttung 6 in den Reformierungsprozeß eingebracht. Dadurch kann der Reformierungsprozeß, d.h. das thermodynamische Gleichgewicht, sowie die Temperatur zusätzlich stark beeinflußt bzw. optimiert werden. Es kann auch eine der Rohrleitung 27 entsprechende Rohrleitung direkt, ohne den Weg über die Verdampferwendel zu nehmen, in die Reaktionszone eingeführt werden.

Die Ein- und Auslässe können je nach Anwendungsfall gewechselt werden, so daß die Strömungswege ihre Richtung umkehren.

Wie schon ausgeführt, können in den obigen Ausführungsbeispielen Brennstoffe verschiedener Arten, wie Erdgas, Benzin, Methanol, Diesel, Flüssiggas oder dgl. reformiert werden. Die Temperaturen, auf die der Katalysator aufgeheizt wird, hängen von der Art des Brennstoffs ab. Beispielsweise beträgt die Temperatur bei Diesel mehr als 600°C, während bei Methanol 300°C ausreichen.

## Patentansprüche

1. Verfahren zur autothermen Reformierung von Kohlenwasserstoffen, bei dem ein im Wesentlichen flüssiger und/oder gasförmiger Brennstoff gemischt mit einem Ausgangsstoff der Reformierung einer einen Katalysator aufweisenden Reaktionszone zugeführt wird, wobei der Katalysator auf einer Temperatur gehalten wird, die eine Kohlenstoffbildung, eine Verkokung oder thermisches Cracken verhindert, wobei der Brennstoff oder die Mischung Brennstoff-Ausgangsstoff mit einer Temperatur kleiner 250°C direkt auf den heißen Katalysator aufgebracht wird und eine Verbrennung und Reformierung gleichzeitig katalytisch durchgeführt wird.

2. Verfahren nach Anspruch 1, **dadurch gekennzeichnet, daß** das entstehende Reformat im Gegenstrom und in wärmetauschender Weise mit mindestens einem Teil des Ausgangsstoffs geführt wird.

3. Verfahren nach Anspruch 1 oder 2, **dadurch gekennzeichnet, daß** zum Starten der Reaktion der Katalysator elektrisch vorgeheizt wird und/oder der Brennstoff für eine kurzzeitige reine Verbrennung durch einen Zündfunken gezündet wird.

4. Verfahren nach einem der Ansprüche 1 bis 3, **dadurch gekennzeichnet, daß** als Ausgangsstoff Luft und/oder Sauerstoff und/oder Wasser und/oder Wasserdampf verwendet wird.

5. Verfahren nach einem der Ansprüche 1 bis 4, **dadurch gekennzeichnet, daß** vorgewärmte oder nicht vorgewärmte Ausgangsstoffe und/oder Brennstoff direkt in den mittleren Bereich der Reaktionszone eingebracht werden.

6. Verfahren nach einem der Ansprüche 1 bis 5, **dadurch gekennzeichnet, daß** der Katalysator abhängig vom Brennstoff auf Temperaturen größer 300°C aufgeheizt und gehalten wird.

7. Vorrichtung zur autothermen Reformierung von Kohlenwasserstoffen mit einem einen Katalysator (4, 5, 6) aufweisenden Reformierreaktor (1), eine zuführungseinrichtung (19) zum Aufdüsen von Brennstoff direkt auf den Katalysator (4, 5, 6) und Mittel zum Halten des Katalysators abhängig vom Brennstoff durch Regelung der zugeführten Stoffe auf einer Temperatur von 300°C oder mehr, wobei der Reformierreaktor (1) unterhalb der zuführungseinrichtung (19) angeordnet ist und die zuführungseinrichtung (19) in einen Mischraum (3) direkt auf den Katalysator gerichtet, hineinragt, in dem der Brennstoff beim Aufdüsen auf den Katalysator mit Ausgangsstoffen der Reformierung mischbar ist.

8. Vorrichtung nach Anspruch 7, **dadurch gekennzeichnet, daß** der Katalysator des Reformierreaktors (1) als mindestens ein mit Katalysatormaterial beschichteter permeabler Festkörper (4, 5) ausgebildet ist.

9. Vorrichtung nach einem der Ansprüche 7 bis 8, **dadurch gekennzeichnet, daß** der Reformierreaktor ein Katalysatormaterial aufweisendes Schüttbett (6) umfaßt.

10. Vorrichtung nach Anspruch 8, **dadurch gekennzeichnet, daß** der permeable Festkörper ein metallischer und/oder keramischer Wabenkörper ist.

11. Vorrichtung nach Anspruch 8 oder 10, **dadurch gekennzeichnet, daß** der permeable Festkörper zwei übereinander liegende getrennte Teile (4, 5) aufweist, wobei der obere Teil elektrisch vorheizbar ist.

12. Vorrichtung nach einem der Ansprüche 8 bis 10, **dadurch gekennzeichnet, daß** der Reformierreaktor (1) von einem Wärmetauscher (2) umgeben ist.

13. Vorrichtung nach Anspruch 12, **dadurch gekennzeichnet, daß** der Wärmetauscher (2) mehrere konzentrisch angeordnete Ringspalten aufweist, in denen mindestens ein Teil der Ausgangsstoffe der Reformierung im Gegenstrom mit dem aus dem Reformierreaktor (1) austretenden Reformat von außen nach innen geführt werden.

14. Vorrichtung nach einem der Ansprüche 7 bis 13, **dadurch gekennzeichnet, daß** die Zuführungseinrichtung eine Düse (19) aufweist, die den Brennstoff auf das Katalysatormaterial des permeablen Festkörpers (4, 5) aufdüst.

15. Vorrichtung nach Anspruch 14, **dadurch gekennzeichnet, daß** die Düse als Zweistoffdüse ausgebildet ist, durch die zusätzlich zum Brennstoff Ausgangsstoff eingedüst wird.

16. Vorrichtung nach einem der Ansprüche 12 bis 15, **dadurch gekennzeichnet, daß** der Wärmetauscher (2) eine Verdampferwendel (24) aufweist, die von den gesamten oder von einem Teil der Ausgangsstoffe und/oder von Brennstoff durchströmt ist.

17. Vorrichtung nach einem der Ansprüche 12 bis 16, **dadurch gekennzeichnet, daß** der Wärmetauscher (2) mit einer Leitung (27) verbunden ist, die einen Teil des Ausgangsstoffs in den Mittelbereich des Reformierreaktors (1) führt.

## Claims

1. Process for autothermal reforming of hydrocarbons, in which an essentially liquid and/or gaseous fuel mixed with a starting material of reforming is supplied to a reaction zone having a catalyst, wherein the catalyst is maintained at a temperature which prevents carbon formation, coking or thermal-cracking wherein the fuel or the fuel-starting material-mixture with a temperature lower than 250°C is applied directly to the hot catalyst and combustion and reforming is carried out catalytically at the same time.

2. Process according to claim 1, **characterised in that** the resulting reformate is passed in counter-current and in heat-exchanging manner with at least some of the starting material.

3. Process according to claim 1 or 2, **characterised in that** to start the reaction the catalyst is pre-heated electrically and/or the fuel is ignited for a brief pure combustion by means of an ignition spark.

4. Process according to one of claims 1 to 3, **characterised in that** air and/or oxygen and/or water and/or steam is used as starting material.

5. Process according to one of claims 1 to 4, **characterised in that** pre-heated or non-pre-heated starting materials and/or fuel are introduced directly into the central region of directly into the central region of the reaction zone.

6. Process according to one of claims 1 to 5, **characterised in that** the catalyst is heated and maintained at temperatures greater than 300°C dependently of the fuel.

7. Device for autothermal reforming of hydrocarbons having a reforming reactor (1) which has a catalyst (4, 5, 6), a supply device (19) for spraying fuel directly onto the catalyst (4, 5, 6) and means for maintaining the catalyst at a temperature of 300°C or more dependently of the fuel by controlling the materials supplied, wherein the reforming reactor (1) is arranged below the supply device (19), and the supply device (19) projects into a mixing chamber (3), directed directly to the catalyst, in which the fuel can be mixed with starting materials of reforming during spraying onto the catalyst.

8. Device according to claim 7, **characterised in that** the catalyst of the reforming reactor (1) is designed as at least one permeable solid body (4, 5) coated with catalyst material.

9. Device according to one of claims 7 to 8, **characterised in that** the reforming reactor encloses a bulk bed (6) having catalyst material.

10. Device according to claim 8, **characterised in that** the permeable solid body is a metallic and/or ceramic honeycomb.

11. Device according to claim 8 or 10, **characterised in that** the permeable solid body has two superposed separate parts (4, 5), wherein the upper part can be pre-heated electrically.

12. Device according to one of claims 8 to 10, **characterised in that** the reforming reactor (1) is surrounded by a heat exchanger (2).

13. Device according to claim 12, **characterised in that** the heat exchanger (2) has several concentrically arranged annular gaps, in which at least some of the starting materials of reforming are passed from outside inwards in counter-current with the reformate emerging from the reforming reactor (1).

14. Device according to one of claims 7 to 13, **characterised in that** the supply device has a nozzle (19) which sprays the fuel onto the catalyst material of the permeable solid body (4, 5).

15. Device according to claim 14, **characterised in that** the nozzle is designed as a binary nozzle, through which starting material is sprayed in addition to the fuel.

16. Device according to one of claims 12 to 15, **characterised in that** the heat exchanger (2) has an evaporator coil (24), through which flows the entire or some of the starting materials and/or fuel.

17. Device according to one of claims 12 to 16, **characterised in that** the heat exchanger (2) is connected to a pipe (27) which passes some of the starting material to the central region of the reforming reactor (1).

## Revendications

1. Procédé de reformage autothermique d'hydrocarbures, dans lequel un combustible sensiblement liquide et/ou gazeux mélangé à une matière de base du reformage est acheminé dans une zone de réaction équipée d'un catalyseur, le catalyseur étant maintenu à une température qui empêche une formation de carbone, une carbonisation ou un cracking thermique, le combustible ou le mélange combustible/matière de base étant amené à une température inférieure à 250°C directement sur le catalyseur chaud et une combustion et un reformage étant effectués simultanément par voie catalytique.

2. Procédé selon la revendication 1, **caractérisé en ce que** le reformat obtenu est guidé en contre-courant et de manière à assurer un échange thermique avec au moins une partie de la matière de base.

3. Procédé selon la revendication 1 ou 2, **caractérisé en ce que**, pour lancer la réaction, le catalyseur est préchauffé par voie électrique et/ou le combustible est activé par une étincelle d'allumage pour une combustion pure de courte durée.

4. Procédé selon l'une quelconque des revendications 1 à 3, **caractérisé en ce que** la matière de base utilisée est l'air et/ou l'oxygène et/ou l'eau et/ou la vapeur d'eau.

5. Procédé selon l'une quelconque des revendications 1 à 4, **caractérisé en ce que** des matières de base.préchauffées ou non préchauffées et/ou un combustible sont introduits directement dans la partie centrale de la zone de réaction.

6. Procédé selon l'une quelconque des revendications 1 à 5, **caractérisé en ce que** le catalyseur est chauffé, en fonction du combustible, à des températures supérieures à 300°C et est maintenu à ces températures.

7. Dispositif de reformage autothermique d'hydrocarbures, comprenant un réacteur de reformage (1) contenant un catalyseur (4, 5, 6), un dispositif d'acheminement (19) destiné à injecter le combustible directement sur le catalyseur (4, 5, 6) et des moyens destinés à maintenir le catalyseur, en fonction du combustible, à une température de 300°C ou plus par le réglage des matières acheminées, le réacteur de reformage (1) étant disposé en dessous du dispositif d'acheminement (19) et le dispositif d'acheminement (19) pénétrant dans une chambre de mélange (3) en étant dirigé directement sur le catalyseur, dans laquelle le combustible, au moment de son injection sur le catalyseur, peut être mélangé directement avec les matières de base du reformage.

8. Dispositif selon. la revendication 7, **caractérisé en ce que** le catalyseur du réacteur de reformage (1) est formé par au moins un corps solide (4, 5) perméable, revêtu du matériau catalyseur.

9. Dispositif selon l'une quelconque des revendications 7 à 8, **caractérisé en ce que** le réacteur de reformage (1) comporte un lit de déversement (6) contenant un matériau catalyseur.

10. Dispositif selon la revendication 8, **caractérisé en ce que** le corps solide perméable est un corps métallique et/ou céramique à alvéoles.

11. Dispositif selon la revendication 8 ou 10, **caractérisé en ce que** le corps solide perméable comporte deux parties (4, 5) séparées superposées, la partie supérieure pouvant être préchauffée par voie électrique.

12. Dispositif selon l'une quelconque des revendications 8 à 10, **caractérisé en ce que** le réacteur de reformage (1) est entouré par un échangeur thermique (2).

13. Dispositif selon la revendication 12, **caractérisé en ce que** l'échangeur thermique (2) comporte plusieurs fentes annulaires concentriques, dans lesquelles est guidée, de l'extérieur vers l'intérieur, au moins une partie des matières de base du reformage en contre-courant avec le reformat sortant du réacteur de reformage (1).

14. Dispositif selon l'une quelconque des revendications 7 à 13, **caractérisé en ce que** le dispositif d'acheminement comporte une buse (19), par laquelle le combustible est injecté sur le matériau catalyseur du corps solide (4, 5) perméable.

15. Dispositif selon la revendication 14, **caractérisé en ce que** la buse est conçue sous forme de buse à deux matières, par laquelle la matière de base est injectée en plus du combustible.

16. Dispositif selon l'une quelconque des revendications 12 à 15, **caractérisé en ce que** l'échangeur thermique (2) comporte un évaporateur hélicoïdal (24), à travers lequel circule tout ou partie de la matière de base et/ou du combustible.

17. Dispositif selon l'une quelconque des revendications 12 à 16, **caractérisé en ce que** l'échangeur thermique (2) est relié à une conduite (27) par laquelle une partie de la matière de base est amenée dans la partie centrale du réacteur de reformage (1).
